# EUROPEAN PATENT APPLICATION

(11) **EP 1 867 338 A1**
(43) Date of publication of application: **19.12.2007**
(21) Application number: 06114741.9
(22) Date of filing: 30.05.2006
(51) Int. Cl.: A61K 38/17, A61P 35/00

(54) **Pharmaceutical composition comprising apolipoproteins for the treatment of human diseases**

(71) Applicant: Université Libre de Bruxelles, 1050 Brussels (BE)
(72) Inventor: Pays, Etienne, 1457 Nil St Vincent (BE); Vanhollebeke, Benoit, 1310 La Hulpe (BE)
(74) Representative: pronovem

(57) **Abstract**

The present invention is related to a pharmaceutical composition comprising an adequate pharmaceutical carrier or diluent and an element selected from the group consisting of:
- apolipoprotein apoL-Ill;
- a pharmaceutically active fragment of this apolipoprotein apoL-Ill;
- a nucleotide sequence encoding this apolipoprotein apoL-Ill or said fragment;
- a vector comprising said nucleotide sequence;
- a cell transformed by said nucleotide sequence or said vector;
- an inhibitor or activator directed against said apolipoprotein apoL-Ill, its fragment or its nucleotide sequence,
- an anti-inhibitor or anti-activator directed against said inhibitor or activator or its encoding nucleotide sequence.

## Description

### Field of the Invention

The present invention is related to an inhibitor directed against apolipoproteins, selected from the group consisting of apolipoprotein (apoL) family (apoL-I to apoL-VI, preferably apoL-III, apoL-II and/or apoL-I).

Another aspect of the present invention concerns a pharmaceutical composition comprising an adequate pharmaceutical carrier or diluent and an element selected from the group consisting of an apolipoprotein and the inhibitor of the invention, preferably, apoL-III, apoL-II, apoL-IV, apoL-V or ApoL-VI inhibitors, an apolipoprotein (preferably apoL-III, apoL-II, apoL-IV, apoL-V or apoL-VI), nucleotide sequences encoding these apolipoproteins, vectors comprising them, cells transformed by these nucleotide sequences and expressing these apolipoproteins or cells transformed by these nucleotide sequences encoding these inhibitors and expressing these inhibitors.

A further aspect of the present invention is related to the use of this pharmaceutical composition for the manufacture of a medicament in the treatment and/or the prevention of various diseases, preferably selected from the group consisting of cancer (through necrosis senescence or apoptosis, (control of programmed cell-death)) inflammation, arteriosclerosis, angiogenesis and regulation of the immune system (modulating the immune response of a patient, especially for the treatment or the prevention of auto-immune diseases), neurological disorders (including schizophrenia) .

### Background of the invention

ApoL-I was identified as a component of a minor subclass of HDL particles in human blood [1]. This protein is encoded by a gene belonging to a multigene family that contains six members organised as a cluster on the 22q13.1 locus [2, 3]. DueLinked to its association with lipoproteins that participate in the transport of cholesterol, apoL-I was, and still is, considered as playing a role in the transport and metabolism of lipids. Some correlations between the serum levels of apoL-I and triglycerides appeared to support this hypothesis [4, 5].

African trypanosomes are flagellated protozoan parasites responsible for major plagues of the continent. In particular, two Trypanosoma brucei subspecies (T. b. Trypanosoma brucei rhodesiense and T. b. gambiense) can grow in humans where they cause a lethal disease termed sleeping sickness. Although morphologically indistinguishable from these pathogens, the T. b. brucei subspecies is readily lyzed by human serum. Studies aiming at understanding the mechanism of resistance of T. b. rhodesiense to this lytic activity allowed the identification of apoL-I as trypanolytic factor 1[6].

The toxic activity of apoL-I on T. b. brucei was recently analyzed into some detail [7]. ApoL-I is targeted to the lysosome of the parasite, following active endocytosis of the carrier lipoproteins [8]. The progressive acidification occurring during endocytosis probably releases the toxin from HDLs and allows its insertion into the lysosomal membrane. ApoL-I was found capable of generating anionic pores in various biological membranes. This activity is responsible for the toxic effect of apoL-I on trypanosomes. It triggers uncontrolled influx of chloride from the cytoplasm to the lysosome, and leads to continuous osmotic swelling of this compartment, followed by and subsequent cell death.

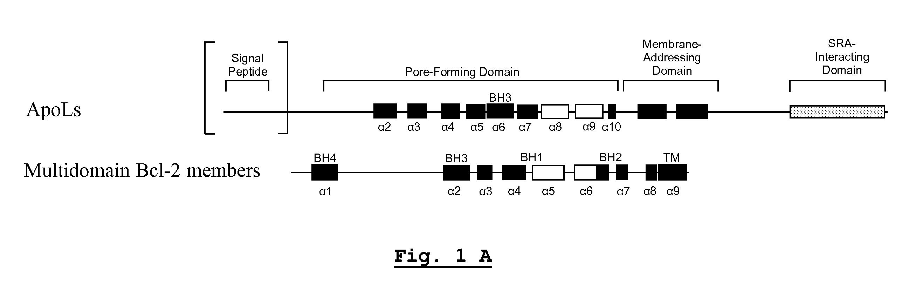

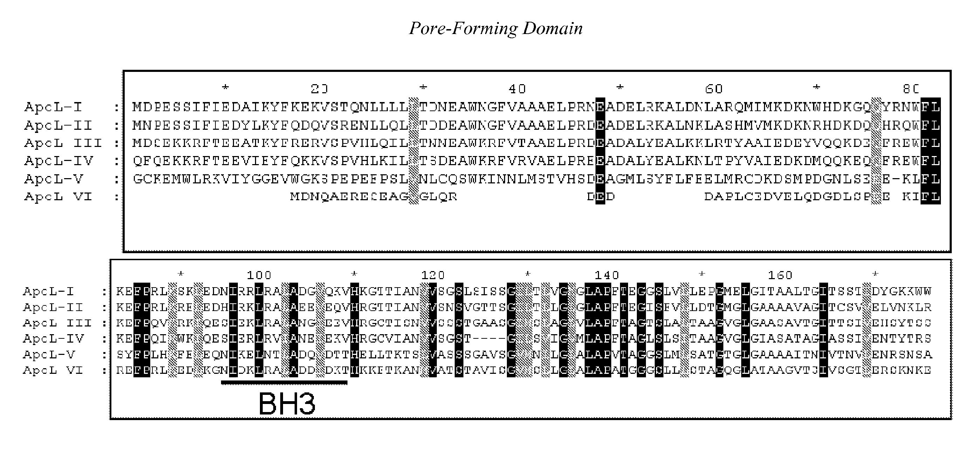

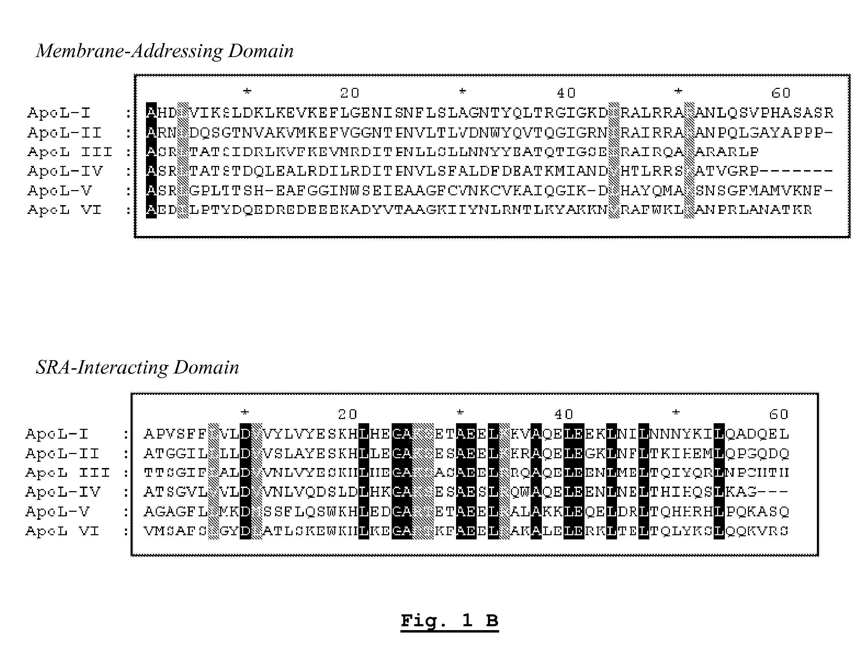

During studies on the trypanolytic activity of apoL-I, experimental data obtained on the basis of model predictions allowed the definition of three distinctive domains in apoL-I [7] (Fig. 1). This protein contains a pore-forming domain whose organisation resembles that of bacterial colicins, diphtheria toxin and the mammalian Bcl-2 family members: a long hydrophobic hairpin surrounded by a bundle of amphipathic alpha helices. As in colicins, the pore-forming domain of apoL-I is unable to reach the target membrane without the presence of an adjacent membrane-addressing domain. This domain consists of a pH-sensitive hairpin bridging two alpha helices. At neutral pH this domain exhibits a hydrophobic surface that would allow the protein to associate with HDLs . The last domain is a long amphipathic alpha helix with a leucine zipper. This helix that is not required for the toxic activity of the protein, on trypanosomes as well as on other cell types.

ApoL-I can be distinguished from the other members of the family due to the presence of an additional sequence encoding an N-terminal signal peptide, probably arisen after tandem duplication and responsible for the secretion of this protein in the serum. The other members do not possess such a signal peptide sequence, and are believed to be intracellular. Sequence analysis predicts them to be targeted to the endoplasmic reticulum (apoL-I,-II, -IV, -VI) or to the cytoplasm (apoL-III, -V) [3], but the cellular localisation of these proteins remains to be determined. In the cases of apoL-III and apoL-IV, alternative splicing generates rare splice mRNA variants that encode a putative signal peptide [2], but none of these proteins has yet been detected in the serum.

Phylogenetic analysis reveals that the apoLs are closely related, although apoL-V and apoL-VI appear to be more evolutionarily divergent [3]. This is paralleled by the relative distance of these two genes from the others in the genomic cluster. ApoL-I was identified only in humans and Gorilla [9, 10]. However, apoL homologues are present in a variety of mammalian species, always organised as a cluster but with a variable number of members (mice have as many as 14 members while rats have only 8). some with Rapid gene duplication makes it difficult to delineate homologues of individual human apoLs outside the primate lineage. Non-mammalian species such as zebrafish also appear to contain members of the family. Based on genomic evidence, apoL-III seems to be the ancestor gene of the apoL-I-apoL-IV cluster [11], and this particular member exhibits the highest homology with apoLs from other organisms. The closest homologue to apoLs is a protein termed verge (Vascular Early Response Gene), which is conserved between mice and humans and may thus represent an early divergent protein that arose from this family [12]. Other distantly related members can be found in Caenorhabditis elegans and Zea mays [7, 12] .

Based on the effect of apoL-I on trypanosomes and its demonstrated anionic pore-forming activity of this protein in various cell types and biological membranes [7].

### Aims of the present invention

The present invention aims to provide a new pharmaceutical composition for improving the treatment and/or the prevention of various diseases affecting humans, especially cancer (through necrosis senescence and/or apoptosis), inflammation, arteriosclerosis, angiogenesis and neurological disorders, including schizophrenia.

The present invention aims also to provide such composition for improving modulation of an immune response of a patient, especially for the treatment and/or the prevention of autoimmune diseases.

### Summary of the invention

A first aspect of the present invention is related to an inhibitor or an activator of apolipoproteins. According to the invention apolipoproteins are molecules, which belong to an apolipoprotein (apoL) family (apoL-I to apoL-VI).

Preferably, said apolipoproteins are human mammal apolipoproteins, preferably human apolipoproteins. These inhibitors or activators are molecules, which interact with these apolipoproteins for modulating their activity.

An example of these inhibitors or activators are antibodies or peptides (preferably monoclonal antibodies or hypervariable specific portion thereof) directed against (to) these apolipoproteins and which are able to modulate (inhibit, reduce or improve) the activity of these apolipoproteins. Preferably, these inhibitors are not the SRA proteins or a portion thereof and do not include inhibitors directed against apoL-I protein.

These inhibitors or activators can be lipids ions or synthetic molecules.

Preferably, these inhibitors or activators are natural peptides obtained from a human sample, which may interact specifically with these apolipoproteins, preferably against apoL-III, but possibly also against apoL-II, apoL-IV, apoL-V and/or apoL-VI.

Another aspect of the present invention is related to the nucleotide sequence encoding the inhibitor or activator peptide, according to the invention or a portion thereof interacting with the apolipoprotein and to the vector comprising this nucleotide sequence.

A further aspect of the present invention is related to a cell transformed by this nucleotide sequence or a vector and encoding and possibly expressing the inhibitor or activator peptide.

The present invention is also related to a method for recovering from a human sample (such as a blood sample) the inhibitor or activator according to the invention and directed against an apolipoprotein, preferably against apoL-III, apoL-I and/or apoL-II. This method comprises the steps of:
- fixing this apolipoprotein or a portion thereof upon a solid support;
- contacting the biological sample with this fixed apolipoprotein or a fragment thereof and obtaining a specific binding between the inhibitor present in the sample and this fixed apolipoprotein protein or its fragment;
- washing the biological sample in order to recover the inhibitor or activator molecule bound to the apolipoprotein or its fragment and,
- possibly eluting the solid support, in order to recover the inhibitor or activator.

The present invention is also related to these mammals, preferably to these human inhibitors molecules recovered by said method and directed against apolipoproteins.

A further aspect of the present invention is related to a pharmaceutical composition comprising a pharmaceutically acceptable carrier or diluent, and an element selected from the group consisting of an inhibitor or activator directed against (to) an apolipoprotein or a fragment thereof (including an inhibitor directed against apoL-I SRA or a fragment thereof included), a nucleotide sequence encoding this inhibitor or activator, a vector comprising this nucleotide sequence, the cell transformed by the nucleotide sequence or the vector, an apolipoprotein (apoL-I to apoL-VI), a nucleotide sequence encoding this apolipoprotein, a vector comprising this nucleotide sequence, a cell transformed by this nucleotide sequence or this vector, or a mixture thereof.

The present invention is also related to the use of the pharmaceutical composition according to the invention for the manufacture of a medicament in the treatment and/or the prevention of various diseases, especially the treatment of cancer (through necrosis, senescence or apoptosis) inflammation, arteriosclerosis, angiogenesis, and neurological disorders including schizophrenia.

The present invention is also related to the use of this medicament for the immune system of a patient in the treatment and/or the prevention of autoimmune diseases.

The present invention is also related to a method of treatment of a mammal subject, including a human patient, which comprises the step of administrating a sufficient amount of the pharmaceutical composition according to the invention for the treatment and/or the prevention of these diseases, or for modulating the immune system.

A preferred embodiment of the present invention is related to the use of apoL-III, possibly in combination with another apolipoprotein, inhibitor or activator of apoliproteins for the manufacture of a medicament for the treatment and/or the prevention of cancer, especially through necrosis senescence or apoptosis of cells, more preferably through necrosis or apoptosis of human blood cells.

Preferably, the molecule according to the invention is used for obtaining an efficient treatment, preferably the destruction of cancer cells through necrosis (senescence or apoptosis) following the formation of ionic channels in the membrane of these cells. Therefore, all the above described apolipoproteins may be used for a similar function.

The original function of apoL might be ionic channels of intracellular membranes. They could play a role in the regulation of organelle and/or cellular or cell volume, or maintenance of membrane potential. One obvious concerned organelle would be the lysosome, as it is the target compartment for apoL-I [7]. Several chloride channels of the CIC family are known to act on the endosomal compartment, but it is clear that other players remain to be discovered, since the absence of endolysosomal CICs did not affect the control of the lysosomal pH and volume [13].

These apolipoproteins, their inhibitors or activators, or their encoding sequences could also be used in gene therapy or cellular therapy, possibly in combination with a suitable vector expressed in a specific cell. Therefore, another aspect of the present invention concerns a vector comprising a nucleotide sequence encoding at least one of these apolipoproteins or one or more inhibitors (or activators) directed against (to) these apolipoproteins.

The vector can be a plasmid, a virus, a cationic vesicle, or any other suitable molecule able to obtain the transformation of a cell, preferably a cancer cell and possibly an expression of the corresponding encoded polypeptide in the cell or by the cell.

Another example of the inhibitor or activator according to the invention could be a nucleotide sequence which is complementary to the nucleotide sequence encoding the apolipoprotein. Examples of such nucleotide sequence are antisense mRNA, SiRNA, miRNA or other interference nucleotide sequences having alternative antisense structural type such as phosphorothiate, Morpholino, PNA (peptide nucleic acid), LNA (locked nucleic acid), and 2'-O alkyl oligo. All these antisense structural types have been used as antisense therapy technologies. Another example of inhibitor or activator nucleotide sequences are ribozymes. Examples of ribozymes are Harpin ribozymes, Hammerhead ribozymes and synthetic ribozymes which may comprise ribozyme activity inhibitors, such as a small molecule inhibitor or Morpholino antisense oligo.

Another aspect of the present invention concerns an anti-inhibitor or anti-activator molecule which is directed against the inhibitor or activator according to the invention and that could be a peptide or a nucleotide sequence (including antisensense sequences or ribozyme sequences), specifically directed against the inhibitor or activator peptide or nucleotide sequence. Preferably for avoiding its binding to an apolipoprotein.

### Brief description of the Drawings

Fig. 1 is representing three distinctive domains of apoL-I.

Fig. 2 represents the BH3 domain in apoL-I to apoL-V involved in pore-forming.

### Detailed description of the invention

In metazoans, apoptosis leads to the selective removal of useless, damaged or dangerous cells from the body, avoiding the abrupt dispersal of intracellular content. This evolutionary conserved process is essential for morphogenesis and tissue sculpting during development and homeostasis throughout life. Apoptosis death executioners are members of the caspase family of proteases, which upon activation cleave vital proteins and activate other proteolytic enzymes [14]. These caspases may act either independently or in concert with another key protein family involved in programmed cell death: the Bcl-2 family [14-16]. This family is composed of both pro- (such as Bid, Bax, Bcl-Xs, Bak, Bad, Bim, Puma) and anti- (such as Bcl-2, Bcl-xL, Bcl-w) apoptotic proteins, and is characterized by the presence of one or several Bcl-2 homology signature motifs (BH1 to BH4). Various proteins containing only the BH3 motif (termed BH3-only proteins) can sense different stress conditions and initiate apoptosis [17]. Through different stimuli (e.g. truncated Bid), pro-apoptotic molecules (Bax, Bak) undergo conformational remodelling that allows their translocation to the mitochondrial membrane, causing the depolarization and permeabilization of this membrane. Cytochrome c and Smac/Diablo are released from the mitochondrion into the cytoplasm, which activates the caspase pathway.

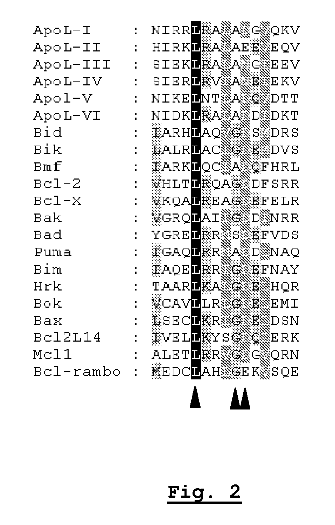

As the Bcl-2 family acts upstream from irreversible cellular damage, they play a pivotal role in deciding whether a cell will die or not. Following the insertion of members of the Bcl-2 family into the mitochondrial membrane different members of this Bcl-family share a colicin pore-forming-like domain [18], and both pro- and anti-apoptotic members have been shown to possess ionic pore-forming activity in vitro [19-22]. Both characteristics, the presence of a colicin-like pore-forming domain and the capacity to conduct ions, have been found and described for apoL-I [7] . ApoLs and Bcl-2 share the same phylogenetic distribution: absence in bacteria and yeast, presence in metazoans. Even though the Bcl-2 proteins family is not present in yeast and bacteria, evidence demonstrated their potential for toxic activity was demonstrated in those organisms [23-25], as was also shown for apoL-I [7]. Moreover, in a screen for novel BH3-only proteins apoL-VI was selected [26]. In fact, the presence of a BH3 domain in helix 6 of the pore-forming domain [7] could characterize the entire apoL family, as shown in Fig. 2. Furthermore, over expression of apoL-VI in cancer cells devoid of p53 led to typical characteristic signs effects of apoptosis such as induction of initiator caspases 8 and 9, and release of cytochrome c and smac/Diablo from the mitochondrion [26]. Significantly, induction of caspases 3 and 9, translocation of phosphatidylserine in the plasma membrane toxic effect of apoL-VI was lost upon deletion of dependent on the presence of the putative BH3 motif.

In accordance with a role in cell survival, a growing number of reports point to significant modification (increase or decrease) of the expression level of apoLs in several cancers, particularly cervical cancer, ovarian cancer and breast cancer [27-30]. Under normal conditions, apoL expression appears to be very low, being undetectable by Northern blot analysis. Upregulation of putative prodeath proteins in cancer cells might look puzzling at first sight. However, the BH3-only protein subset of the Bcl-2 family is implicated in the first events of the apoptotic pathway, as they act as sensors of different death stimuli. Cancer cells are still in contact with death stimuli, but in some way they escape them. Therefore, it is possible that this escape occurs downstream from the steps targeted by apoLs, accounting for the upregulation of these sensors. Interestingly, apoLs are also upregulated in some virus infected cells [30, 31], and apoL-I was shown to be the most upregulated gene during replicative senescence [32, 33]. Although the biological significance of senescence is not fully understood, it may be seen as a process of escape from apoptosis. One of the strategies, along with apoptosis and quiescent-like growth arrest, to counteract abnormal cell proliferation [34].

ApoL transcripts are present in a wide variety of organs [2, 3, 11]. As apoL-I, apoL-II and apoL-III gene expression is restricted to the endothelium lining blood vessels [11, 35], this wide expression pattern might be explained by the presence of endothelial cells in various organs. In endothelial cells, which are primary targets of cytokine-induced cell death, apoLs are strongly induced by the pro-inflammatory cytokines TNF-α [11, 35] and IFN-γ [36]. Similarly, the related protein verge, which is also restricted to the endothelium, is overexpressed by TNF-α [12]. IFN-α and IFN-β regulate apoLs transcripts levels [30, 31], while apoL-III expression activates the NF-κB pathway [37]. ApoL-I transcripts were also detected in macrophages, placenta and neurones of the prefrontal cortex [3, 11, 35, 38]. The high expression level in the placenta [3] might be related to extensive angiogenesis [39] that involves not only cellular proliferation, but also regression of endothelial cells during involution of capillary networks, probably through an apoptotic process [40, 41]. Finally, microarray data demonstrated selective expression of apoL-III (and to a lesser extent apoL-I and apoL-II) in immune T and B cells. Taken together, these observations raise the possibility that apoLs are involved in processes linked to cytokine-induced cell death, particularly in the endothelial and immune systems. In lymphocytes, apoptosis can be induced by the anti-inflammatory glucocorticoid hormones [42], but necrotic cell death pathways also appear to be important to control the decline of the immune response and the elimination of autoreactive cells [43]. Although these processes are still poorly understood, they are both blocked by Bcl-2 [42, 43]. Given the probable presence of the Bcl-2 interacting motif BH3 in apoLs, these proteins are possible candidates for this function. In support of this hypothesis, microarray data indicate that glucocorticoids modulate the expression of apoL-III in Jurkat T lymphocytes [44].

The intracellular localisation of apoLs is totally unknown. A candidate target site is the lysosome, given the optimal preference of apoL-I activity of apoL-I at acidic pH and the localization of this protein in the lysosomal membrane of trypanosomes [7], but predictions based on the sequence suggest a localisation in the endoplasmic reticulum [3]. Obviously, this localisation could be multiple and influenced by the environmental conditions, as seen in the case of Bcl-2, which in addition to the mitochondrion, can prevent membrane leak in the lysosome [45] and in the endoplasmic reticulum [46].

The classical target organelle of apoptosis is the mitochondrion, but increasing evidence reveals that cell death (apoptosis, parapoptosis) can be triggered from alternative organelles such as the Golgi, the endoplasmic reticulum or the lysosome [15, 17, 43]. In particular, a variety of inducers, including the well-known p53 protein, trigger an apoptotic process where permeabilization of the lysosomal membrane precedes activation of Bax and depolarization of the mitochondrial membrane is preceded by a perturbation of the lysosomal membrane [47-49]. The release of the lysosomal cysteine proteases cathepsins into the cytosol appears to play a key role in this pathway, as cathepsin D can trigger activation of Bax [50], and inhibitors of cathepsin those proteins preventted further apoptosis tic events [50, 51]. Although cathepsins released from lysosomes can generate truncated versions of Bid that are able to induce activation of Bax [52], it would seem that in vivo, activation of the mitochondrial pathway by lysosomal permeabilization is Bid-independent [50, 53, 54]. Even very well-known apoptotic inducers such as p53 were shown to act partially through the lysosome. The factors responsible for permeabilization of the lysosomal membrane are still unknown, although In addition, Bax was recently reported to has been shown to partially localise into the lysosomal membrane of apoptotic fibroblastse [49], while in mouse hepatocytes the Bax-activator Bid appeared to be involved in lysosomal permeabilization induced by TNF-α [55]. However, in T lymphocytes exhibiting lysosome-mediated apoptosis, no trace of activated Bax was detectable in lysosomes, and depletion of Bax did not impede lysosomal membrane permeabilization [50]. Similarly, in mouse embryonic fibroblasts the absence of Bax did not prevent normal lysosome-initiated apoptosis [48, 51]. The factors involved in lysosomal permeabilization could contain the interactive BH3 motif, and, since this process appears to be pathway is inhibited by Bcl-2, demonstrating cross-talk between the two pathways [45]. Interestingly, high levels of apoL-III transcripts characterize the cell types (T lymphocytes and endothelial cells) where TNF-α + IFN-γ-induced apoptosis operates through both mitochondrial and lysosomal pathways, while in other cell types (HeLa and HEK293), the lysosomal pathway is absent [54]. Moreover, the apoptotic effect induced by overexpression of apoL-VI in p53-depleted cells was found to be Bid-independent [26], similarly to what occurs in lysosome-mediated apoptosis triggered by TNF-α +IFN-γ in HUVE (Human Umbilical Vein Endothelial) cells [54]. One characteristic of the lysosomal pathway is activation of caspase 3 that is Bid-independent. Interestingly, the apoptotic effect induced by over expression of apoL-VI in cancer cells was found to involve caspase 3 cleavage without Bid cleavage, suggesting activation of the lysosomal pathway. Altogether, these observations suggest a possible involvement of apoLs in the lysosome-mediated pathway of apoptosis.

In the case of apoL-I, HDL sequestration might prevent this toxin to target any cell type when circulating in blood vessels, but after release from the carrier particles in the endocytic compartment of trypanosomes, physiological levels of apoL-I (around 8 pg/ml) readily lyze the parasites [6]. Interestingly, Staphyloccocus aureus L-forms are sensitive to the particular minor HDL subfraction that contains apoL-I [56], suggesting that different pathogens might be sensitive to the same toxin. The general toxicity exhibited by apoL-I on all various cell types tested (bacteria, yeast, trypanosomes and eukaryotic cells) [7] suggests a role of this protein in human innate immunity.

It is probable that not only pathogens, but also human endocytic cells take up HDL-bound apoL-I. Obviously these cells must cope with apoL-I toxicity, raising the general question of the control of apoLs. Under normal conditions apoLs seem to be expressed at very low levels. However, the viability of endothelial cells does not appear to be affected by the strong overexpression of apoL-I, apoL-II and apoL-III induced by TNF-α treatment [11]. Therefore, the toxic potential of apoLs must clearly be controlled in these cells.

The ultimate decision of a cell to die or survive generally depends on the ratio between pro- and anti-apoptotic stimuli. Particularly crucial is the ratio between pro- and anti-apoptotic Bcl-2 family members, as anti-apoptotic members are known to neutralise their toxic counterparts by physical interaction through the BH3 motif [16, 17] Therefore, we propose that pro-survival proteins like Bcl-2 could interact with apoLs to block their activity.

In addition, the studies on the resistance of some African trypanosomes to the lytic effect of apoL-I offers another very seducing possibility. T. b. rhodesiense and T. b. gambiense are resistant to the toxin and can grow in humans, causing sleeping sickness. The mechanism of resistance of T. b. rhodesiense has been elucidated [6, 57]. This parasite expresses a protein (Serum Resistance-Associated protein, or SRA) that confers resistance through physical interaction with the C-terminal helix of apoL-I. Removal of this helix from apoL-I retained its full lytic activity of apoL-I, both on trypanosomes and on bacteria [6, 7]. Thus, the SRA-interacting helix is totally dispensable for the activity of apoL-I. Despite this observation, the C-terminal helix was found to be the most conserved domain between the various apoL family members, whether from humans (Fig. 1) or from other mammals. Altogether, these findings suggest that T. b. rhodesiense might have acquired resistance by neutralisation of apoL-I through its cognate, conserved control domain. In other words, mammalian "SRA-like" proteins may be involved in the natural control of apoL toxic activity through interaction with the C-terminal helix of these proteins.

The absence of apoLs from lower eukaryotes, coupled to the wide distribution and remarkable expansion of the apoL family between mammalian species, point to an important function linked to the physiology of complex organisms. Based on the available information, apoLs represent a new family of intracellular ionic channels, particularly in lymphocytes and endothelial cells. These proteins are induced by inflammation, and could be could be involved in cytokine-induced apoptosis in those cell types. Biological processes possibly concerned by apoLs include cancer, atherosclerosis, angiogenesis and regulation of the immune system. In addition, microarray studies have revealed that apoL-I, apoL-II and apoL-IV transcripts are highly increased in prefrontal cortex of schizophrenic patients [38].

## Claims

1. A pharmaceutical composition comprising an adequate pharmaceutical carrier or diluent and an element selected from the group consisting of:
- apolipoprotein apoL-III;
- a pharmaceutically active fragment of this apolipoprotein apoL-III;
- a nucleotide sequence encoding said apolipoprotein apoL-III or said fragment;
- a vector comprising said nucleotide sequence;
- a cell transformed by said nucleotide sequence or said vector;
- an inhibitor or activator directed against said apolipoprotein apoL-III, its fragment or its nucleotide sequence,
- an anti-inhibitor or anti-activator directed against said inhibitor or activator or its encoding nucleotide sequence.

2. The composition according to claim 1, which further comprises an element selected from the group consisting of :
- apolipoprotein apoL-II;
- apolipoprotein apoL-I;
- pharmaceutically active fragment of this apolipoprotein apoL-II and/or apoL-I;
- nucleotide sequences encoding said apolipoprotein apoL-II, apoL-I or their fragments;
- vector(s) comprising said nucleotide sequence(s);
- cells transformed by said nucleotide sequence(s) or said vector(s); and
- inhibitor(s) or activator(s) directed against said apolipoproteins apoL-II, apoLIII, their fragment, their nucleotide sequence and/or,
- anti-inhibitor(s) directed against said inhibitor(s) or activator(s) or encoding nucleotide sequence(s).

3. The composition according to claim 1 or 2, wherein the inhibitor or activator is a peptide inhibitor.

4. The composition according to claim 1 or 2, wherein the inhibitor or activator is an antisense nucleotide sequence or a ribozyme.

5. The composition according to any of the preceding claims, wherein the inhibitor or activator is a (monoclonal) antibody or a specific hypervariable portion thereof.

6. The composition of claims 1 to 3, wherein the inhibitor is the SRA polypeptide or an active fragment thereof.

7. Use of the pharmaceutical composition according to any of the preceding claims for the manufacture of a medicament in the treatment and/or the prevention of a disease selected from the group consisting of cancer, inflammation, arteriosclerosis, angiogenesis and/or neurological disorders including schizophrenia and autoimmune diseases.
